Europäisches Patentamt

European Patent Office

Office européen des brevets

(11) Publication number: **0 249 832**
**A2**

# EUROPEAN PATENT APPLICATION

(21) Application number: **87108177.4**

(22) Date of filing: **05.06.87**

(51) Int. Cl.⁴: **A61B 19/08**

(30) Priority: **09.06.86 US 873038**

(43) Date of publication of application:
**23.12.87 Bulletin 87/52**

(84) Designated Contracting States:
**BE DE FR GB IT LU NL SE**

(71) Applicant: **ARGON MEDICAL CORP.**
**214 East Corsicana**
**Athens Texas(US)**

(72) Inventor: **Holden, Richard Wesley II**
**Apartment 209 401 South Carroll**
**Athens Texas(US)**
Inventor: **Finney, Michael Joseph**
**1117 Oval Drive**
**Athens Texas(US)**
Inventor: **Guest, Robert Luther, Jr.**
**Route 4 Box 4715**
**Athens Texas(US)**

(74) Representative: **Vossius & Partner**
**Siebertstrasse 4 P.O. Box 86 07 67**
**D-8000 München 86(DE)**

(54) **Drape absorbent pad with cufflike dam.**

(57) An improved surgical drape (10) includes a cuff-like dam (22) which prevents spillage of fluids which are not absorbed by the absorbent material of the drape (10). The cuff-like dam (22) is comprised of a folded portion along the edge (20) of the drape. The folds (18) are retained by adhering them in place along the edge (20) of the drape (10). (Fig.)

# DRAPE ABSORBENT PAD WITH CUFF-LIKE DAM

## Background of the Invention

The present invention relates to an improved surgical drape.

In many medical procedures a drape incorporating an absorbent section is used to provide a sterile operating field and to prevent spillage of body fluid and/or liquids used in the procedure. Frequently, these fluids amass so rapidly they spill from the absorbent section, prior to being absorbed. Accordingly, it would be desirable to have an improved surgical drape which would prevent such spillage from occurring.

In accordance with the present invention, a a cuff-like dam is provided on one or more edges or in one or more strategic locations of the surgical drape in order to thereby impede the flow of spilled fluids and thereby allow them to be absorbed.

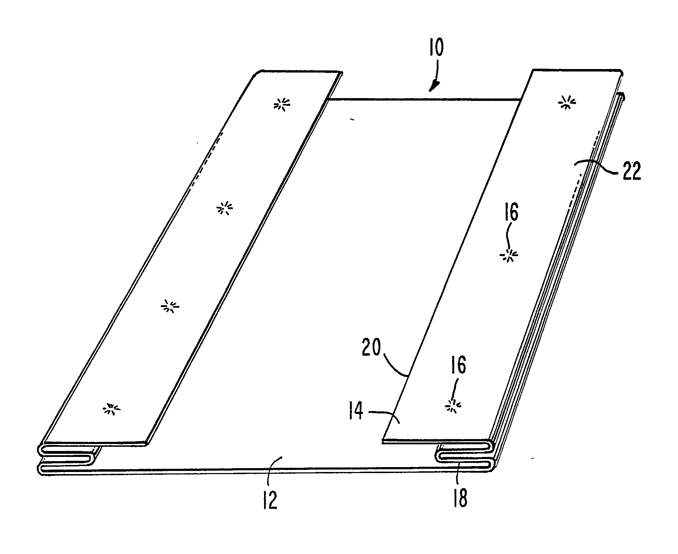

The sole Figure of the Drawing illustrates an improved surgical drape made in accordance with the present invention.

Referring to the sole Figure of the Drawing, a surgical drape 10, manufactured in accordance with the present invention is shown. The surgical drape 10 is comprised of a folded piece of absorbent material to which cuffs 22 have been formed. The cuffs 22 may comprise folds 18 which serve to keep the edge 20 of the material away from the main body of the absorbent 12, thereby forming a cuff-like dam 14 which serves to catch any flow.

The formation of the cuff-like dam 14 may be accomplished by adhering additional absorbent (or non-absorbent) material to the main portion 12 or by folding the existing material along an edge 22, as shown. The fold 18 is maintained in the material by means of adhesions 16 which serve to keep the folds 18 in place.

As will be understood by those skilled in the art, the present invention can be comprised of any combination of folds, adhesions, and/or additional material capable of providing a flow impediment.

## Claims

1. An improved surgical drape of the type comprised of an absorbent material adapted to overlie a patient during a surgical procedure, wherein the improvement comprises a cuff-like dam formed on at least one edge of said surgical drape.

2. The improved surgical drape of Claim 1 wherein the cuff-like dam is formed by folding an edge of said drape back upon itself.

3. The improved surgical drape of Claim 2 wherein said folded edge is adhered to the main portion of said surgical drape in order to retain said fold in place.

10

22

16

20

14

16

12

18